# EUROPEAN PATENT SPECIFICATION

(11) **EP 2 707 080 B1**
(45) Date of publication and mention of the grant of the patent: **23.08.2017**
(21) Application number: 12726203.8
(22) Date of filing: 08.05.2012
(51) Int. Cl.: A61M 37/00, B29C 41/36, B81C 99/00, B29C 41/42, B29C 41/22, A61K 9/00

(54) **METHOD FOR FABRICATING A MICRONEEDLE**
VERFAHREN ZUR HERSTELLUNG EINER MIKRONADEL
PROCÉDÉ DE FABRICATION D'UNE MICROAIGUILLE

(30) Priority: 09.05.2011 GB 201107642
(43) Date of publication of application: 19.03.2014
(73) Proprietor: University College Cork, Cork (IE)
(72) Inventor: MOORE, Anne, Cork (IE); VRDOLJAK, Anto, Cork (IE)
(74) Representative: D Young & Co LLP
(86) International application number: PCT/IB2012/052283
(87) International publication number: WO 2012/153266

(56) References cited:
- EP-A1- 2 213 284
- EP-A1- 2 283 809
- WO-A2-2008/011625
- WO-A2-2008/130587
- WO-A2-2010/041873

## Description

### FIELD OF THE INVENTION

The present invention relates to a method for fabricating microneedles for percutaneous delivery of drugs, vaccines or other materials. The method involves at least partially pre-filling a needle-forming cavity of a mould with a solvent, prior to application of a microneedle-forming composition. Once the composition has been applied, the solvent and composition may mix, for example as a result of diffusion, the solvent may then be removed and the resulting microneedle demoulded.

### BACKGROUND TO THE INVENTION

The default delivery route for most of the current vaccines and biological products as well as many drugs is injection using hypodermic needles and syringes. Although well established and routinely used, this application route has several drawbacks. Hypodermic needle injection is painful, it requires involvement of trained personnel, generates hazardous sharps-waste and in addition many of the products require cold-chain storage and distribution.

An alternative to hypodermic-needle based delivery systems that is useful for some drugs is the transdermal patch. However, in most cases these systems are applicable for delivery of low molecular weight and lipophilic molecules only which can spontaneously traverse the outermost layer of the skin, the stratum corneum.

Various microneedle-based delivery platforms were suggested as a replacement for current hypodermic route in cases where simple transdermal patch is ineffective, such as those described in Banga (2009) Expert Opin Drug Deliv 6(4): 343-54; Prausnitz et al. (2008) Nat Biotechnol 26(11): 1261-8 and Donnelly et al. (2010) Drug Deliv 17(4): 187-207. Microneedles are solid or hollow micron scale projections ranging in height typically 50-700µm that pierce the stratum corneum and thereby enable or facilitate transport of drugs and vaccines across the skin barrier in cases where simple transdermal administration is ineffective.

The concept of microneedle arrays for administering drugs through the skin was first proposed in 1970s, such as in U.S. Patent No. 3,964,482. Since then a number of methods for production of solid, hollow or dissolvable microneedles have been proposed. In recent years several methods describing fabrication of sugar or polymer microneedles were proposed such as those in U.S patents 6,451,240 and 6,945,952, WO2002/064193 A2; WO2008/13058 A2; Sullivan et al. (2008) Advanced Materials 20(5): 933-938; Raphael et al. (2010) Small 6(16): 1785-1793; Lee et al. (2011) Biomaterials 32(11):3134-40.

WO 2008/011625 describes a device for sustained delivery of drug across or into a biological barrier, such as skin. The device may include a base substrate which comprises a drug dispersed in a matrix material; and one or more microneedles extending from the base substrate, wherein the one or more microneedles comprise a water-soluble or water-swellable material, wherein the one or more microneedles will dissolve or swell following insertion into the biological barrier, providing a transport pathway for the drug to pass from the base substrate into the biological barrier.

Polymeric microneedles seem to offer certain advantages compared to other types of microneedles. This is well demonstrated in several reports on usage of dissolvable microneedles made of biocompatible sugars or polymers for vaccine delivery with recent reports describing delivery of inactivated influenza viruses being the most technically and immunologically advanced to date (Raphael et al. (2010) Small 6(16): 1785-93; Sullivan et al. (2010) Nat Med 16(8): 915-20). Most current methods rely on the production of degradable microneedles by filling the formulation into a negative (female) mould having microdepressions which define the surface of the microneedles and subsequent drying and/or hardening of the material. However, the filling of the liquid formulation in microcavities of the mould is not a spontaneous process due to the microscale dimensions of the cavities, surface tension and often high viscosity of the liquid formulation being filled.

Various methods have been employed in order to fill the needle cavities of the mould with the desired formulation. Most often used approaches are (a) applying a centrifugal force on the mould with the formulation deposited on top of the mould, such as described in U.S. patent 2011/0028905 A1, (b) pressurizing the mould with the formulation deposited on top of the mould, such as described in WO2008/130587 A2, and (c) vacuuming the mould with the formulation deposited on top of the mould, such as described in Monahan et al. (2001) Anal. Chem. 73, 3193 - 3197. These methods allow uniform filling of the wells with extremely small volumes, which might be difficult to achieve using other filling methods such as direct microinjection, inkjet printing, micropipetting, or using a picospritzer, as discussed in Grayson et al. (2004) PROCEEDING OF THE IEEE, 92(1), 6-21.

However, fabrication methods for the production of dissolvable microneedle arrays described to date have certain disadvantages. One of the major drawbacks in described methods of making dissolvable microneedle patches is the need for application of large volumes of formulation onto microneedle mould in the filling step where only a fraction of the volume used actually fills the needle holes while the rest remains unused. Although theoretically the excess of the formulation remaining on the surface of the moulds may be reused, such recycling approach may imply compliance issues according to Good Manufacturing Practice (GMP) code when the process is scaled to an industrial level. This problem is especially emphasized in the methods employing vacuum-filling as the formulation solvent might partially evaporate during the filling step and change its composition. Other methods such as centrifugation-based methods are challenging in an aspect of scaling up to an industrial level. In addition, methods described to date generally require multiple filling steps if they are to be used for filling the whole needle body volume with any formulation which loses volume upon drying, such as all water-based formulations. This occurs when the volume of formulation placed initially into the needle cavity decreases due to water (or any other solvent) loss as the result of solvent evaporation. This will result in only partially filled needles and the filling process would need to be repeated to fill the remaining of the needle cavity. If repeated, however, the filling step could result in recurring partial dissolving/drying of formulation already delivered in the first step. Such repeated dissolving/drying could damage some sensitive active substances such as proteins and viruses.

Another potential disadvantage of the current fabrication methods as they are described is the necessity to use a backing layer in which microneedles are embedded. This backing layer is normally used to fix and connect individual microneedles into a compact array. Although in certain applications the backing layer can also contain an active substance, it is usually pharmacologically irrelevant and only a design necessity playing no active role in the actual drug-delivery process. However, making a backing layer adds to the complexity of the fabrication process and quality control and hence increases cost. Therefore methods of fabrication which omit the requirement for a backing layer could simplify production process.

Yet another limitation of most of the current mould-based methods is that the whole microneedle patch effectively has to be uniform i.e. all the needles are the same in composition. However, the generation of heterogeneous patches containing individual microneedles that are composed of different materials and containing different active components may be beneficial in certain applications. For example, several active components may not be mutually compatible in the same formulation or may require different formulations for stabilization, or a desired reaction between them may only be required upon delivery in the skin, e.g., an enzyme-substrate interaction. Also, in the case of some drug/vaccine formulations where components cannot be mixed into a single solution this method would overcome the requirement for multiple components to be filled into separate vials as each drug/vaccine could be included on the same vaccine delivery device in discrete individual microneedles.

A theoretical approach to circumvent at least some of the described drawbacks of the current method would be depositing the liquid formulation of interest directly into each needle cavity of the mould, as discussed in theory in WO2008/130587 A2. However, given the micron scale precision needed for such dispensing devices and respective moulds as well as surface tension and viscosity of formulations issues, this approach does not seems to be a viable option at the current state of technology. To the best of the present inventors' knowledge, no such functional device has been made to date for the above reasons.

Apart from the mould-based methods for production of dissolvable microneedles, several non-mould based approaches have been described, such as in Lee et al. (2011) Biomaterials 32(11):3134-40. However, these methods rely on the use of high temperatures during the fabrication process (>100 °C) which is incompatible with fragile biopharmaceutical components such as proteins and viruses. Alternatively, methods have been described which aim to enhance the drying or curing the drug-containing formulation (EP228309), however these methods still rely on previously described filling methods, such as centrifugation or other physical forces.

### SUMMARY OF ASPECTS OF THE INVENTION

The present inventors have developed a new method that overcomes the problem of partial mould filling, without the need for applying a centrifugal force on the mould, pressurizing the mould or vacuuming the mould. They have found that if the mould is pre-filled with solvent, prior to application of a microneedle-forming composition, the composition properly fills the mould, producing a complete microneedle.

Thus, in a first aspect, the present invention provides a method for fabricating a microneedle using a mould (2) having a needle-forming cavity, characterised in that the method comprises the steps of (a) at least partially filling the needle-forming cavity with a solvent (1), (b) applying a microneedle-forming composition (3) to the needle-forming cavity such that the composition is brought in contact with the solvent (1), (c) allowing the solvent and microneedle-forming
composition to mix as a result of diffusion, (d) removing the solvent and (e) demoulding the microneedle.

The method may be used for fabricating a microneedle with a needle body formed of two composition materials, by using a method which comprises the following steps:
(a) at least partially filling the needle-forming cavity with a first solvent,
(b) applying a first microneedle-forming composition to the needle-forming cavity such that the composition is brought in contact with the solvent, wherein the first microneedle-forming composition is applied in an amount to partially fill the needle cavity following solvent removal;
(c) allowing the first solvent and first microneedle-forming composition to mix as a result of diffusion;
(d) removing the first solvent;
(e) at least partially filling the remaining needle-forming cavity with a second solvent,
(f) applying a second microneedle-forming composition to the needle-forming cavity such that the composition is brought in contact with the second solvent;
(g) allowing the second solvent and second microneedle-forming composition to mix as a result of diffusion;
(h) removing the second solvent; and
(i) demoulding the microneedle,
thereby forming a microneedle with a body made of two different composition materials.

The solvent may be filled into the mould by spraying atomized droplets of solvent directly into the needle-forming cavity.

The microneedle-forming composition may be applied to the needle-forming cavity in an amount that exceeds the volume of the cavity upon solvent removal, such that a disk of dry material is formed around the microneedle base upon solvent removal.

The microneedle-forming composition may be dried at ambient temperature.

The solvent may be removed by evaporation.

In a first embodiment of this aspect of the invention the microneedle-forming composition forms a dissolvable material following drying, such that when the microneedle is applied to the skin, or other tissue, of a subject it dissolves.

In connection with this embodiment, the microneedle-forming composition may comprise an active substance, such that when the microneedle dissolves upon application to the skin, the active substance is delivered into the underlying tissue of the subject.

The dissolvable material may be or comprise of one or a combination of materials selected from the following group: polymers, carbohydrates, cellulosics, sugars, polyols or alginic acid or a derivative thereof.

The dissolvable material may comprise one or a combination of materials selected from the following: polyvinyl alcohol (PVA), polyvinyl pyrrolidone (PVP), raffinose, sucrose, trehalose, dextran, glycerine, CMC and sodium alginate.

The solvent used for dispersing the dissolvable material may, for example, be water, C2-C8 alcohol, or an organic solvent, or a mixture of solvents

The active substance may be a therapeutic, prophylactic or diagnostic agent.

The active substance may be a drug or vaccine.

The active substance may be selected from the following group: an antibody, a live or inactivated virus or viral vector, a bacterium, protein, glycoprotein, lipid, oligosaccharide, polysaccharide, nucleotides, oligonucleotides, DNA or RNA.

The active substance may be thermolabile

The dissolvable material may comprise a vaccine adjuvant.

When the method is used for fabricating a microneedle with a needle body formed of two composition materials, the first and second microneedle-forming compositions may comprise the same or different active substances.

When the method is used for fabricating a microneedle with a needle body formed of two composition materials, the first microneedle-forming composition material may form a microneedle tip with high mechanical strength and second microneedle-forming composition may form a below-tip portion of low mechanical strength.

In a second aspect, the present invention provides a method according to the first aspect of the invention for forming a microneedle array, by using a mould having a plurality of needle-forming cavities, such that a plurality of microneedles are fabricated.

The method may be used for forming a heterogeneous microneedle array, by using a plurality of different microneedle-forming compositions, each composition being applied to a subset of the needle-forming cavities.

When the method is used for fabricating a microneedle with a needle body formed of two composition materials, the first and/or second microneedle-forniing compositions may be delivered successively or simultaneously on different microneedle cavities of the same mould thus forming a heterogeneous microneedle array.

The microneedle and/or array may be demoulded by adhering to an adhesive surface applied on top of the filled mould and pulling the microneedles out of the mould.

For example, flexible adhesive tape suitable for application on human and/or animal skin may be used for demoulding.

Also described herein is a microneedle. The microneedle may be fabricated by a method according to the first aspect of the invention.

Also described herein is a microneedle array. The microneedle array may be fabricated by a method according to the second aspect of the invention.

Within the array, each needle may be independent and separated from other needles by an area of adhesive. This is a result of the fabrication method, where each microneedle is formed discreetly in the mould and stands individually, such that when the adhesive is applied they are transferred individually on to the adhesive. This configuration is distinct from other microneedle array types where all the microneedles are embedded in a backing layer.

The microneedle array may be heterogeneous in the sense that it comprises at least two subsets of microneedles having different compositions. The microneedles of a given subset may be clustered together on the array, to form "patches" of microneedles of a given composition.

Also described herein is a method for delivering an active substance to a subject, which comprises the step of applying an array as described above which comprises the active substance dispersed in at least part of the microneedle body to a subject, such that the active substance is delivered to the underlying tissue of the subject. The array may, for example be applied to the skin, such that it pierces the stratum corneum of the subject.

Also described herein is a device comprising a microneedle or microneedle array as described above.

Also described herein is a kit for use in a method according to
the first aspect of the invention, which comprises a microneedle-forming composition and one or more of the following: (a) a microneedle-forming mould, (b) apparatus for precise delivery of composition material on to cavities of the mould, (c) drying chamber and (d) suitable adhesive tape for demoulding.

The method of the present invention has several advantages over the previously described methods. For example:
(i) the method allows complete filling of the microneedle mould, thus producing complete microneedles which are sharp enough to pierce a tissue of the body tissue, such as the stratum corneum, to access the underlying tissue of a subject.
(ii) the method avoids the need to apply of large volumes of formulation onto microneedle mould in the filling step where only a fraction of the volume used actually fills the needle holes while the rest remains unused;
(iii) the method enables microneedle arrays to be made in a single filling step without the need for recycling of formulation material;
(iii) the method facilitates preparation of heterogeneous microneedle arrays;
(iv) the method avoids repeated dissolving/drying steps and the use of high temperatures associated with some previous approaches, which are unsuitable for thermolabile active substances
(v) the method allows the use of a separate, rather than an integral backing layer (in which microneedles are embedded) which simplifies the production process;
(iv) the method is simple, easy to scale up and has increased potential to be GMP compliant.

### DESCRIPTION OF THE FIGURES

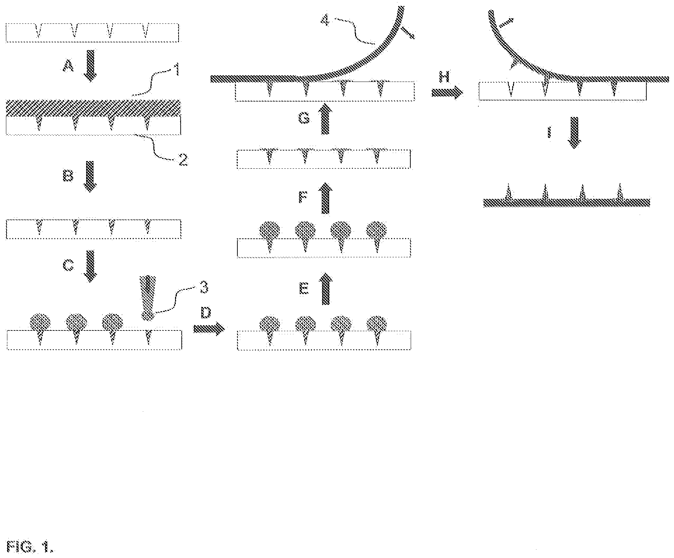

**Fig. 1****.** Schematic diagram of dissolvable microneedle array fabrication process. (A) Water (1) was applied to a PDMS mold (2) under vacuum or by spraying. (B) Excess water was removed from the surface using sharp blade. (C and D) The concentrated drug solution was applied directly on top of needle cavities (3). (E) Concentration of drug solution was equilibrated in the upper bulb and microneedle cavity as a result of diffusion between highly concentrated formulation in the bulb and water in the cavities. (F) The drug solution was dried. (G) Flexible adhesive tape (4) was applied on top of the mould to adhere to needle bases and lifted (H) giving (I) an array of drug-filled dissolvable microneedles ready for application.

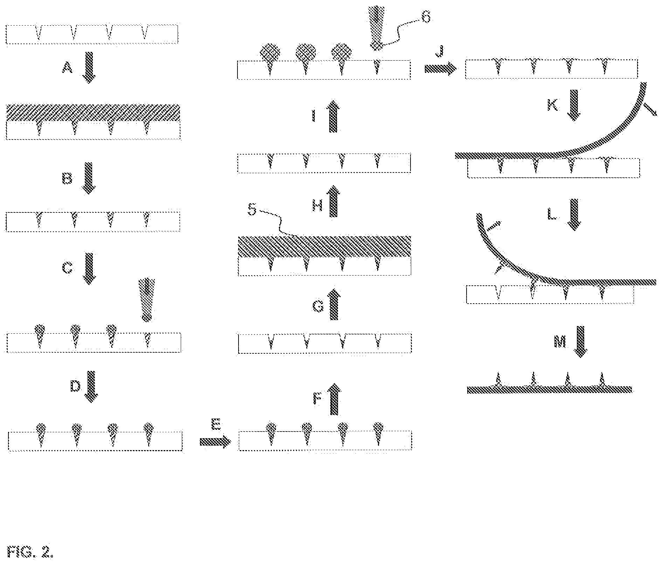

**Fig. 2****.** Schematic diagram of dissolvable microneedle array fabrication process with formulation concentrated in the needle tips. (A) Water was applied to a PDMS mold under vacuum or by spraying. (B) Excess water was removed from the surface using sharp blade. (C and D) Small amount of concentrated drug solution in water was applied directly on top of needle cavities. (E) Concentration of drug solution was equilibrated in the upper bulb and microneedle mould as the result of diffusion between highly concentrated formulation in the bulb and water in the cavities. (F) The drug solution was dried to give formulation concentrated in the needle tips. (G) Second solvent (96% ethanol) was applied to a mold and (H) excess is removed using sharp blade. (I and J) Solution of polyvinylpyrrolidone (PVP) in 96% ethanol (6) was added on top of dry formulation and dried. (K) Flexible adhesive tape was applied on top of the mold to adhere to needle bases and lifted (L) giving an array of dissolvable microneedles with drug filled in the needle tips and hard support base made of PVP (M).

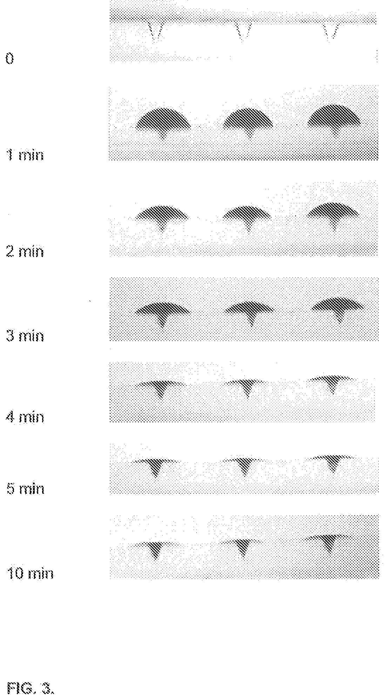

**Fig. 3****.** Drying of drug formulation dropped on PDMS mould prefilled with water. Formulation consisting of 50% trehalose (w/v) and methylene blue delivered on top of PDMS mould prefilled with water dries in approx. 5 min at ambient temperature. Second picture also shows that diffusion of formulation from upper bulb into microneedle cavity seems to be complete even after 1 min (needle tips are blue confirming that diffusion equilibrated concentration in the bulb and microneedle cavity).

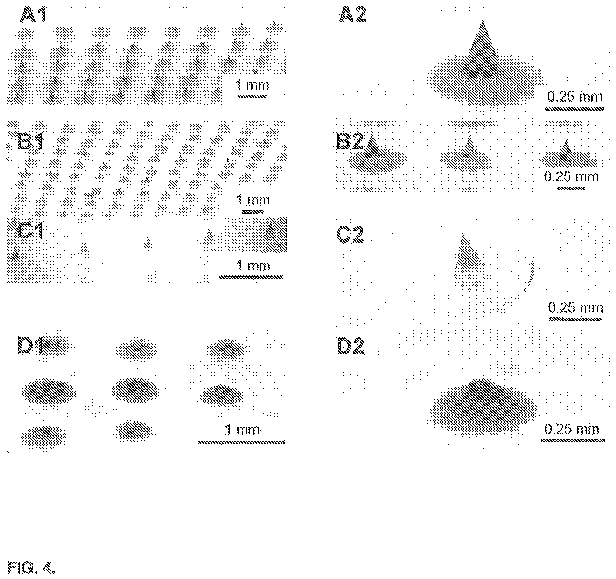

**Fig. 4****. Examples of dissolvable microneedles.** Homogeneous microneedle array (A1) and individual needle (A2). Heterogeneous array containing needles made of two different formulations (B1) and magnified part (B2). Microneedle array with formulation concentrated in the needle tips with transparent PVP base (C1-C2). Incomplete needles formed if prefilling of needle cavities with water was omitted from fabrication procedure (D1-D2).

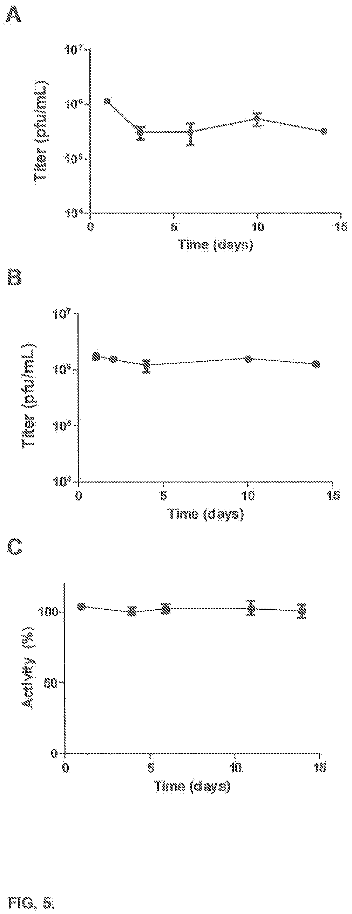

**Fig. 5****.** Stability of adenovirus (AdV), Modified Vaccinia Ankara virus (MVA) and lysozyme embedded in microneedles during 14 days at ambient temperature. AdV coding for mCherry fluorescent protein (A), MVA coding for RFP protein (B) and lysozyme (C) were embedded in dissolvable microneedles made of trehalose (AdV) or trehalose/PVA (MVA and lysozyme) and left at ambient temperature for 14 days. Y-axis represents log PFU equivalent units for AdV and MVA or % activity for lysozyme with error bars showing standard deviation.

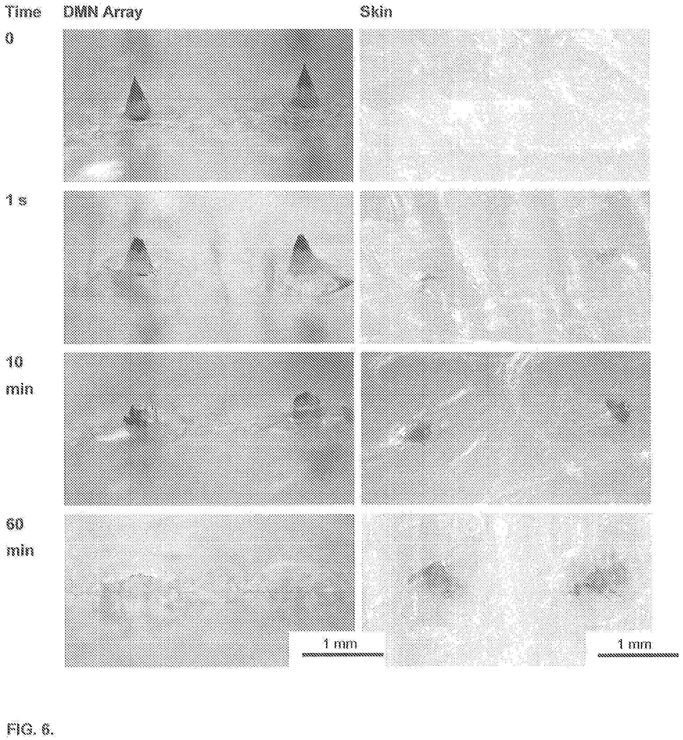

**Fig. 6****.** Kinetics of dissolution of dissolvable microneedles with formulation concentrated in the needle tips. Arrays with 500um tall needles were fabricated with needle tips made of trehalose with the addition of Congo red dye and base made of PVP with the addition of methylene blue dye. Arrays were then applied onto cadaver pig skin and left for 1 s, 10 min and 60 min after which they were imaged using light microscope.

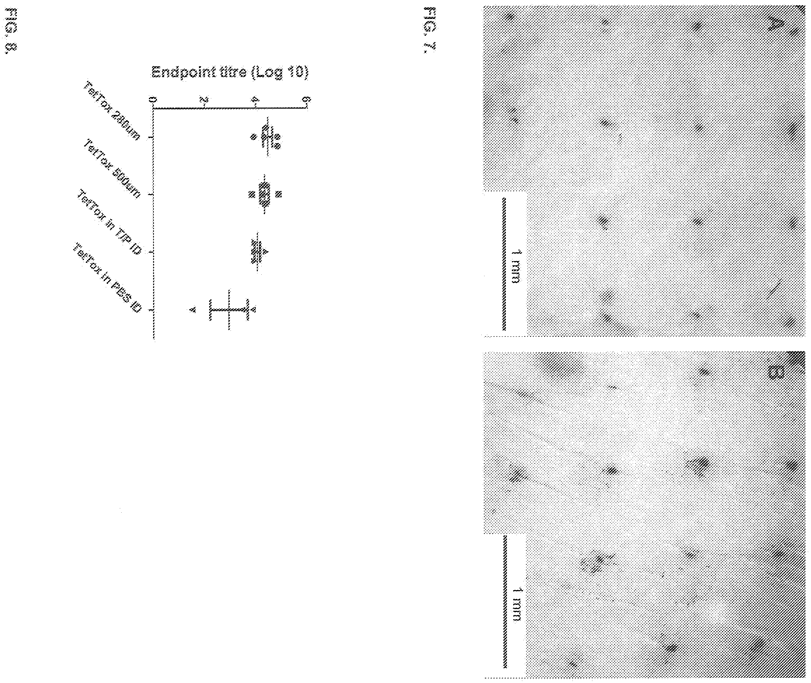

**Fig. 7****.** Skin-transfection using dissolvable microneedle arrays with AdV-β-gal (A) or MVA-β-gal (B). Microneedle arrays with 280µm long needles containing either AdV-β-gal or MVA-β-gal were applied onto freshly excised pig and later examined for β-galactosidase expression
**Fig. 8****.** Antibody induction to tetanus toxoid antigen due to vaccination with antigen in dissolvable microneedle arrays. Tetanus toxoid protein antigen was formulated with trehalose and PVA ('T/P') and incorporated into arrays with 280µm or 500µm tall microneedles. These arrays were applied to mouse ears (1 to each ear of each mouse). As controls, tetanus toxoid in formulation ("TetTox in T/PVA ID") or in PBS ("TetTox in PBS ID) was injected as a liquid by the intradermal route. Serum anit-tetanus toxoid antibody titres were determined 3 weeks post-immunization by ELISA.

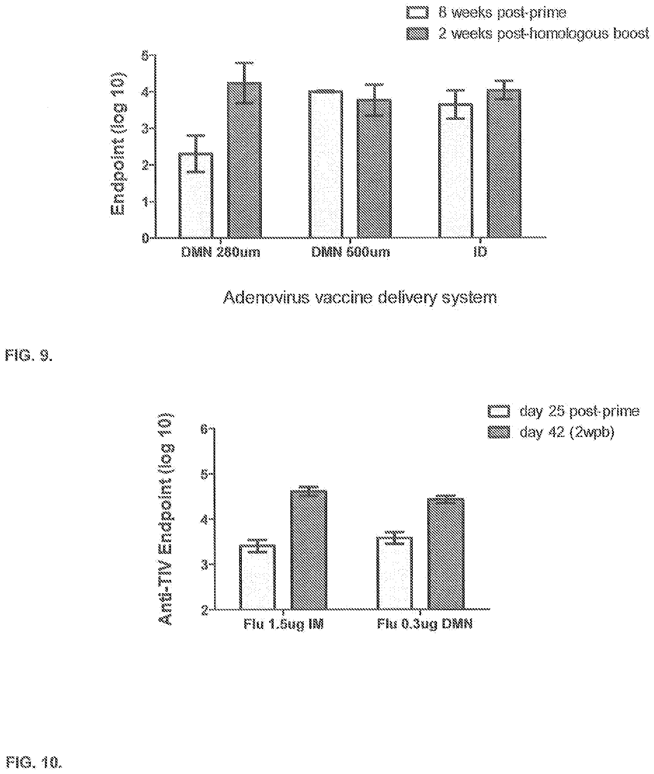

**Fig. 9****.** Antibody induction by a recombinant adenovirus virus vector that induces antibody responses to the encoded *Plasmodium yoelii* antigen MSP-1 (Draper et al (2009) Cell Host Microbe 5, 95-105 and (2008) Nat Med 14, 819-21). Live recombinant adenovirus was formulated with trehalose and incorporated into arrays with 280µm or 500µm tall microneedles; termed 'DMN280µm' and 'DMN500µm' respectively. These arrays were applied to mouse ears (1 to each ear of each mouse). As a control, recombinant adenovirus was injected as a liquid by the intradermal route (ID). On day 86 post-prime, all mice were re-immunized with the same vaccine regime. Serum antibody titres to the recombinant transgene, MSP-1 were determined 8 weeks after the first immunization and 2 weeks after the boosting immunization by ELISA.
**Fig. 10****.** Antibody induction by a clinically available seasonal influenza vaccine. Seasonal trivalent inactivated influenza vaccine ('TIV') recommended for use in the 2010/2011 northern hemisphere immunization campaign was formulated with trehalose and PVA and incorporated into arrays with 500µm tall microneedles. A total of 1% of the full human dose, equivalent to 0.15µg of each hemagglutinin antigen (HA) was incorporated into each array. These arrays were applied to mouse ears (1 to each ear of each mouse), resulting in the delivery of 0.3 µg of each HA to each animal, equivalent to a total of 2% of the full human doses. As a control, 10% of a full human dose of TIV (equivalent to 1.5µg of each HA) was injected as a liquid by the intramuscular route (IM). On day 28 post-prime, all mice were re-immunized with the same vaccine regime. Serum antibody titres to the vaccine antigens were determined at day 28 and day 42 by ELISA.

### DETAILED DESCRIPTION

### Biological barriers

The microneedle devices disclosed can be applied for the transport of materials into or across biological barriers such as skin (or parts of skin), mucosal tissue, cell membranes or other biological membranes in humans, animals or plants. Typical application of disclosed devices is for delivery of materials into or across the skin. Mammalian skin can be subdivided into three layers; the stratum corneum (SC); in humans this is 10-20µm in depth, the viable epidermis which is 50-100µm in humans and the dermis which is 1-3mm in humans. The outermost layer, the stratum corneum is composed of closely packed dead keratinocytes embedded in a highly organized intercellular lipid matrix that forms a barrier that is impermeable to microbes and large molecules such as vaccine antigens. It is this outer layer that restricts successful unassisted transdermal delivery.

### Microneedles

The microneedle devices disclosed here include arrays or patches used for delivering an active substance through the stratum corneum of the skin or for withdrawing or sampling fluid from the skin or interstitia. It consists of a substantially flat base, on which is mounted a plurality of microneedles where not necessarily all the microneedles are made of the same materials nor they necessarily carry the same active substance. Upon application to the skin, the microneedles extend through the stratum corneum into the epidermis or deeper into the underlying dermis where the active substance is released or the tissue is monitored or sampled.

Methods of application of microneedles onto skin may vary from single rolling motion to pressing the patch substantially vertically on to the skin with or without the use of special devices, essentially as described in Haq et al. (2009) Biomed Microdevices 11:35-47.

The microneedle has sufficient mechanical strength to penetrate the stratum corneum.

The plurality of microneedles is arranged onto an array or patch either in a single row or as a two dimensional array. The microneedle patch or array may be provided as a single patch with the dimensions of, for example, of between 3-15 mm x 3-15mm. Alternatively a carrier sheet may contain larger number of patches which subsequently may be cut into individual patches of the required size. An individual array may contain 10 to 1000 or more microneedles, for example 25-100 per patch.

The shape of the microneedles is designed to permit successful penetration into the skin. Examples include conical- or pyramidal- shaped microneedles, such as described in Wilke et al. (2005 Microelectronics Journal 36:650-656). Usually a sharp needle tip is required for successful skin penetration. Such shapes are well known in the field.

### Methods of fabrication of dissolvable microneedles

The microneedle devices disclosed herein are made by controlled filling of the cavities corresponding to the negative of the microneedles with the biocompatible material to form the microneedles and removing them in a controlled manner to form a microneedle array ready for application. The whole procedure of making the microneedle array can be performed at an ambient temperature or lower thus making it suitable for use with thermo sensitive substances.

### Microneedle templates

Microneedle master templates are used here for the fabrication of negative (female) moulds having microdepressions which define the surface of the microneedles. Microneedle master templates can be made from the variety of materials. Suitable materials of construction include silicon, silicon dioxide, pharmaceutical grade steel, titanium, gold, nickel, iron, tin, chromium, copper and alloys of these metals, polymers such as polycarbonate, polymethacrylic acid, ethylenevinyl acetate, polyesters. Other biodegradable polymers such as lactic acid and glycolic acid polylactide, polyglycolide, polylactide-co-glycolide as well as polyurethanes and other biodegradable polymers may be used. Other materials such as any of the monosaccharides, disaccharides or polysaccharides can be used as well. As in this invention master template is used for the manufacturing of the female mould only and not for the application to the skin, master arrays do not necessarily posses the rigidity usually needed for the application to skin. This allows that master moulds are made from a wider range of materials not normally possessing high rigidity.

Microneedle master templates consist of the plurality of microneedles which may have a length between 50 and 1000µm. The microneedles may have an aspect ratio (height to diameter at base) of at least 3:1 to at least 1:1 or lower. Suitable shapes are conical and pyramidal types of needles where needle diameter decreases with the distance from the base ending in a sharp tip. Other possible microprojection shapes are shown for example in WO2003/024518.

### Moulds

The female moulds used to form microneedles by methods disclosed here can be made from a male master microneedle array using a variety of methods and materials. The suitable materials include for example ceramic materials, silicone rubber, wax, polyurethane, polydimethylsiloxane (PDMS) or other materials which can faithfully take and keep the negative form of the master needle template.

One way of making moulds is by casting the appropriate liquid material over a male master microneedle array. Such materials may dry and harden thus keeping the negative form of the master array. Polydimethylsiloxane and polyurethane are examples of materials suitable for this method of making moulds and commonly used for this process.

Another way of making moulds is from materials which melt at the elevated temperature allowing them to be cast over the master template. After cooling such materials preserve the negative shape of the master template. Alternatively, the male master microneedle array can be pressed onto the soften materials to make negative array. Various waxes and thermoplastics are examples of the materials suitable for this method of making moulds.

Other methods of making microneedle moulds include direct drilling the cavities into mould material, usually by the use of lasers, reactive ion etching methods or electrostatic discharge, depending on the mould material.

Moulds may be reusable or single-use type. Optionally, moulds may be sterilized prior to use using known sterilization techniques such as autoclaving or gamma radiation. Choice of the sterilization method depends on the mould material.

### Micromoulding

Microneedle arrays may be made by micromoulding, by providing a mould having a microdepression which defines the surface of the microneedle, filling the microdepression with moulding material and moulding the material to form a microneedle. The active substance(s) can be included in the composition of the moulded microneedles.

The method of the present invention may comprise the following steps:
(a) providing a mould with cavities corresponding to the negative of the microneedles,
(b) filling the cavities with water or other solvent,
(c) applying the concentrated formulation containing material of interest individually on top of each needle cavity and in contact with solvent already in the cavity,
(d) spontaneous mixing and diffusion between the delivered formulation and the solvent previously filled into the cavities,
(e) removing the solvent and demoulding the microneedles, for example by applying the adhesive tape on top of the mould and pulling the whole array of microneedles out of the mould.

An additional step may be included to concentrate the material of interest in the needle body only, or part of the needle body only, with the remaining of the body and supporting disk being made of a different material.

The microneedles may be formed of a biodegradable polymer at an ambient temperature.

A schematic description of a method in accordance with the invention is given in the Fig. 1.

The prefilling of the mould cavities with water or other solvent may be achieved for example by submerging the mould under the water or solvent and vacuuming the system using an appropriate pump, as described in Monahan et al. (2001) Anal. Chem. 73: 3193 - 3197.

Alternatively, needle cavities can be filled with water or other solvent by spraying the solvent directly into the mould using appropriate spraying apparatus, such as a Schlick nozzle or equivalent, or atomizing the solvent by ultrasonic device, or by other suitable means. The droplets being delivered on the mould range from a fog-like spray to fine droplets. The size of the droplets should be small enough to enter the tips of the microneedle mould without forming air bubbles. The average size of the droplets being delivered onto the mould and into the cavities may be for example less than 15, less than 10 or less than 8 microns in diameter.

The excess of water or other solvent used to fill the cavities remaining on the mould surface can be removed by scraping the surface using steel blade, rubber scrapper or blowing the excess of the solvent using the compressed air blowing to the surface at a low angle, for example at an angle less than 30 degrees to surface.

Solvent used to fill the moulds may be optionally cooled to slow down evaporation. Solvent may be cooled to the temperature lower than ambient, for example at 4-8 °C or lower.

Alternatively, moulds can be placed onto the actively cooled surface to slow down evaporation.

Formulation in the form of concentrated solution containing material of interest is then applied on top of each needle cavity individually in a form of a drop which needs to get into direct contact with the solvent already present in the needle cavity.

The formulation can be disposed on the wells by various means. In one embodiment, formulation is delivered manually with the aid of a precise pump, for example stepper-motor driven syringe pump or pump used in standard High Pressure Liquid Chromatography Systems (HPLC), connected to a thin capillary or needle at an output end. Flow is usually in the range of 1-10µL/min for manual application, depending on the microneedle size. Suitable capillaries are made of glass, silicon, steel, polytetrafluoroethylene (PTFE), flourinated ethylene propylene (FEP), polyether ether ketone (PEEK) or other inert materials. Alternatively, stainless steel hypodermic needles can be used, for example 31G needles. Inner and outer capillary/needle diameters are not critical for the method operation as the capillary/needle does not need to enter the mould cavities but the formulation is instead deposited on top of needle wells, as depicted on Fig. 1. Routinely used are capillaries with the inner diameter of, for example, 100-300µm and outer diameters of, for example, 200-500µm. Capillaries or needles used should be suitable for delivery of formulation in the form of individual drops diameter of which may be smaller, equal or larger than the needle cavity opening. In one embodiment, the diameter of a drop of delivered formulation is larger than the diameter of microneedle cavity resulting in the microneedle body coupled with the surrounding ring made of the same material. In another embodiment, formulation is deposited in the form of a drop having diameter equal or smaller than the cavity opening. In this case formulation does not get into direct contact with the mould but with the water/solvent filled into cavities only. This may further result in the microneedles in which active substance is filled in only the part of the microneedle, as discussed further below.

In one embodiment of the invention precision of the delivery is controlled manually using a magnifier.

In another embodiment, formulation can be delivered onto needle wells in an automated manner using the systems developed for putting down a number of small drops onto substrates in a regular pattern. A variety of such instruments are readily available commercially, for example, from BioDot, Inc. (Irvine, California) or using jet dispensers. Suitable devices consist of a head movable in either two or three dimensions, a reservoir of liquid, a pre-dispensing zone and an opening into the pre-dispensing zone. The liquid is actively delivered onto the mould surface either by a non-contact method where drops of formulation are ejected onto the surface from the distance or by "touch off" methods where the liquid formulation first makes a bridge from the head to the mould surface before being detached from the dispensing head. Dispensing head can be single-channel delivering one drop of formulation at a time or multi-channel delivering two or more drops of formulation at a time. If the number of channels equals the number of wells on the mould the whole array may be developed in a single dispensing step.

Formulation being delivered onto the needle cavity openings does not need to be the same for all microneedles on the array. Different formulations having different composition may be delivered on the same array. This can be achieved by either dispensing formulations sequentially onto respective wells using a single channel or by dispensing different formulations simultaneously using dedicated dispensing channels.

The exact volume of the formulation being delivered depends on the concentration of the formulation, microneedle volume and the microneedle type (needle with the formulation in the whole volume or in the needle tip only). In one embodiment of the invention where the entire microneedle volume contains the active substance needs to be produced, the volume of the drop delivered onto each microneedle cavity is larger than the volume of the microneedle. Exact volume is calculated taking into account the total mass concentration of the formulation being delivered. Typically, the volume of the formulation being delivered will be such that after solvent removal the amount of the dry residue is sufficient to fill the needle cavity. In one embodiment, the volume of the dry residue is even larger than the microneedle volume forming a disk around the microneedle cavity. This disk being formed is important for the needle stability once the needle is transferred onto the adhesive tape and applied onto skin. The disk serves as a wide base support preventing microneedle from flipping during insertion into skin. The diameter of the supporting disk may be for example 150%, 200% or 400% of the needle base diameter or more than that. Fig. 4A1-A2 shows a microneedle with the formulation in the whole needle volume and the disk formed around the needle base.

In another embodiment, the volume of the liquid formulation being delivered is chosen so that after solvent is removed the volume of the dry formulation is less than the volume of the microneedle cavity. In this case an additional step is required to make the remaining of the needle body and stabilizing disk, as explained below.

The diameter of the supporting disk built around the needle base is a function of surface characteristics of the mould, surface tension and contact angle between the formulation being delivered and the mould and the volume of delivered formulation. Larger delivered volume will generally result in a larger supporting disk. Lower surface tension between the formulation being delivered and the mould will generally also result in a disk having larger diameter. Surface tension of the formulation may be altered by the addition of surfactants such as polysorbate, glycerol oleate and sodium dodecyl sulfate. Alternatively, mould material with higher or lower hydrophobicity may be used to alter the disk diameter or the surface properties of the mould may be altered to make it more wettable, for example by methods described in WO 2008/130587 A2.

After the delivered formulation comes into contact with the solvent already present in the microneedle cavity, diffusion will eventually equilibrate the concentration of the active substances throughout the cavity and delivered formulation above the cavity. Solvent from the cavity diffuses into the upper concentrated drop of formulation while the substances from the highly concentrated formulation diffuse into the solvent in the microneedle cavity. Simultaneously, solvent starts to evaporate resulting in the decrease of the volume of the formulation drop above the cavity. Depending on the formulation composition and active substance(s) being used, this process may be performed at ambient conditions or accelerated by placing the mould under vacuum with or without addition of desiccant. The solvent removal process may be performed at ambient temperature, for example at 22-25 °C, or higher, or lower than ambient temperature, depending on the formulation and active substance being used. Duration of the solvent removal process is for example between 10 min and 10 hrs, depending on the formulation being used and the needle design, with taller and larger needles requiring longer process and possibly application of a vacuum.

The drying process may be such that the volume of the initial drop of formulation decreases due to water evaporation ending in the microneedle well filled with the dry formulation. The volume and the concentration of the formulation used may be chosen so that the volume of the dry content upon drying is sufficient to fill the needle cavity and to form a supporting disk around the needle base with a diameter larger, for example, approximately 3 times larger, than the needle base diameter.

In an embodiment of the invention, microneedles in which active substance is contained in the part of the needle body only may be prepared. An example of such a method is shown schemitically in Figure 2. Using this method the amount of the formulation being delivered in the formulation delivery step in this method (Fig. 2, step C) is generally smaller than the volume normally used in the previously described method. Concentration of the delivered formulation in this method may be the same or lower than the concentration of a formulation used for making microneedles with the supporting disk all made of the same material. Generally, concentration and volume of the formulation being delivered onto each well is chosen so that upon solvent removal the volume of the dry residue is less than the volume of the microneedle cavity. During solvent removal formulation will retract below the surface level of the microneedle cavity ending in the dry formulation concentrated in only one part of the needle body. The volume occupied by the dried formulation may be for example in the range of 5-95% of the microneedle body volume. The remaining of the needle body (if any) and supporting disk are generally made of the different material(s) than those used for making the needle tip. The material used to make the rest of the needle body and the supporting disk may be chosen so that it is soluble in at least one solvent in which the first materials(s) exhibit poor solubility. For example, if the needle tip is made from formulation consisting of trehalose the rest of the needle body may be made of PVP dissolved in ethanol in which trehalose is insoluble. This way, attaching the needle base onto the needle tip will not dissolve the tip and disturb the active substance embedded in the tip. Optionally, the second material may also contain the same or other active substance. Optionally, a third or more layer may be added to the microneedle mould to build up a multi-layered microneedle.

The making of the rest of the needle body and the supporting disk is performed generally by the same procedure as the making of the tip (Fig. 2, steps G-K). The mould is filled with the second solvent, excess is removed and the second formulation is added on top of the needle well and allowed to dry resulting in a microneedle in which the first substance is concentrated in the tip area followed by the support and the disk made of the second material.

Most of the current dissolvable microneedle methods require formation of an additional backing layer in which microneedles are fixed. Microneedle arrays made by the method of the invention do not require addition of a backing layer. Microneedles are demoulded from the mould using a suitable adhesive tape in a single step. Suitable adhesive tape is placed on top of the mould containing dry microneedles and surrounding supporting disks. The size of the adhesive sheet may be larger than the size of the array and exceeds the exterior perimeter of the array for at least 1mm or more. An even pressure is applied on the adhesive sheet and the mould using for example finger tip or a suitable tool, for example rubber roller. The disks surrounding microneedle bases and the base of the microneedle adhere to the adhesive tape. The whole array is then demoulded by pulling the adhesive tape with attached microneedles off the mould. Adhesive tape may be suitable for application on human and animal skin, for example 3M(TM) Single Coated Polyester Medical Tape 1516, or similar. The adhesive tape used may be suitable for use on human and animal skin as in that case demoulded array is essentially ready for application without addition of any backing layer. Arrays of microneedles arranged in the described manner have the advantage over the existing arrays as each needle is separated from other needles by an area of adhesive tape. In this way, upon insertion into skin each needle will be surrounded by an area of skin attached firmly onto the adhesive tape. This will result in constant elastic pressure onto each individual needle tip ensuring that needles stay inserted in the skin while dissolving. This prevents needles from tipping out of the skin during dissolving due to possible skin movement.

After demoulding, microneedle arrays arranged on an adhesive tape may be further dried if necessary. Again, this may be performed at ambient conditions or accelerated by placing the array under vacuum with or without addition of desiccant, at ambient temperature, for example at 22-25 °C, or higher, or lower than ambient temperature. Duration of the optional drying step may be 30 min or longer.

For some formulations placing microneedle arrays under vacuum in the presence of dessicant may be used for long-term storage.

### Packaging

In one embodiment of the invention, a plurality of microneedle arrays placed on the same adhesive sheet may be cut into individual arrays and placed into individual packaging. Packaging may be then hermetically sealed and may contain a desiccant to ensure that microneedles retain low moisture content.

Optionally, the whole described process of preparation moulds and formulation to final packaging may be performed using known aseptic and sterilization techniques to ensure sterility of the final product and compliance with GMP regulations.

### Materials of construction

The microneedle arrays described herein may be made at least partly from a material which dissolves when the array is applied to the skin and is in contact with moisture in the skin.

Suitable materials for the production of dissolvable microneedle arrays include any biocompatible, biodegradable or bioerodible polymers, carbohydrates, cellulosics, sugars, sugar alcohols, polyols or alginic acids or a derivative thereof. Suitable materials for the production of microneedle arrays compatible for human or veterinary use include any biocompatible polymers, carbohydrates, cellulosics, sugars, sugar alcohols, polyols or alginic acids or a derivative thereof that are generally regarded as safe (GRAS) or are approved for clinical use in humans or animals.

Suitable formulations may contain only one component or they can be mixtures of more than one component blended in any suitable ratio. Examples include the use of sugars such as trehalose or sucrose, and polymers such as polyvinyl alcohol (PVA) or PVP, alone or in combination.

In addition to main components, suitable formulations for manufacturing of microneedles may optionally include one or more surfactants and/or stabilizing agents, such as amorphous glass-forming sugars.

Also in addition to main components and because the described devices penetrate human skin, one or more pharmaceutically acceptable substances exhibiting antibacterial characteristics may be included in the formulation, such as thiomersal, meta cresol and benzalkonium chloride.

Suitable formulations may be chosen based on the desired dissolution rate *in vivo.* It is well known in the art the kinetics of degradation or dissolution of various polymers, for example, PLGA, in tissue. Additionally, different formulations may be used in different layers of the microneedle that dissolve at different rates in the tissue, thereby permitting pulsed-release of the active material. Alternatively, a slowly dissolving formulation in one part of the microneedle, or the microneedle array, may be used to monitor interstitial fluid, whereas a second or subsequent formulation(s) may release quickly to deliver an active material.

Upon application on skin, microneedles may dissolve completely or only partially, depending on the materials used for fabrication, needle length, duration of exposure on the skin, skin characteristics and thickness. The exact parameters of fabrication and application of the microneedle arrays will be chosen so as to ensure delivery of the active substance to the underlying tissue with appropriate kinetics of release and/or dissolution.

Solvents used for dissolving the formulation may be water, alcohols such as ethanol, propanol, butanol and mixtures thereof. Other suitable non-aqueous solvents include hydrocarbons, esters, ethers, ketones, lactones, nitriles, amides and mixtures thereof. Suitable solvents are compatible with the mould material and result in minimum residual levels in the final, dried microneedle array.

### Active substances to be delivered

The active substance(s) being delivered into skin using microneedles may comprise a therapeutic substance, such as a drug or a vaccine.

The active substance(s) used may be thermolabile.

The term "active substance" used herein refers to any substance with potential therapeutic, prophylactic as well as diagnostic properties when administered to humans, animals or birds, including ex vivo applications. Examples include proteins and peptides such as growth factors, nucleic acids and smaller molecules such as antibiotics, steroids, anaesthetics, antiviral agents.

The active substance to be delivered using microneedles may be a vaccine. The term "vaccine" used herein refers to any prophylactic composition for the prevention of a disease or a therapeutic composition for the treatment of an existing disease.

The term "treatment" used herein means the delivery of the vaccine to a subject suffering from an existing disease in order to lessen, reduce or improve at least one symptom associated with the existing disease and/or to slow down, reduce or block the progression of the disease.

The term "prevention" used herein means the administration of the vaccine to a subject not suffering from the target disease and/or to a subject not yet exhibiting symptoms of the acquired target disease to prevent or impair the cause of the disease (e.g. infection) or to reduce or prevent development of at least one symptom associated with the disease.

A vaccine may comprise a single or multiple components including but not limited to a whole organism vaccine, such as live, killed or attenuated pathogen; a subunit vaccine comprising only a part of a pathogen, or a peptide or protein derivable from such organisms comprising one or more antigenic epitope(s) and adjuvant(s); a nucleotide sequence, such as a RNA or DNA molecule coding a peptide or polypeptide comprising an antigenic epitope(s) and/or adjuvant(s).

The vaccine formulation may include a vector enabling or enhancing the delivery of such a nucleotide sequence to a target cell, such as a plasmid, viral vector, bacterial vector or a yeast vector. Viral vectors include, for example, adenoviral vectors (AdV), adeno-associated viral vectors, herpes viral vectors, retroviral vectors including lentiviral vectors, baculoviral vectors and poxvirus vectors.

Delivery vectors may comprise recombinant (genetically modified) vectors. Viral vectors may be viable, attenuated or replication impaired vectors, such as Modified vaccinia virus Ankara (MVA), adenovirus or semliki forest virus vectors.

### Microneedle device applications

The microneedle devices disclosed can be applied for the transport of materials into or across biological barriers in humans, animals or plants.

A microneedle device as described herein should to be simple to fabricate and to use and may be suitable for self-administration without requiring any special skills. This embodiment may include a microneedle array arranged on an adhesive tape which is pressed onto clean part of the skin and left for certain amount of time until microneedles are dissolved and active substance(s) released into skin. After the application, adhesive tape is peeled off the skin.

Depending on the intended use, microneedle arrays may be engineered to release the active substance(s) relatively quickly, for example within minutes, or to extend release to a longer period, for example one or more days.

### Kits

Also described herein are kits for use in the methods of the present invention.

The kit may comprise a formulation for manufacturing microneedles.

The kit may also comprise a microneedle-forming mould; formulation delivery apparatus; drying chamber; and suitable adhesive tape.

The kit may also comprise an active substance for mixing into a formulation for forming a dissolvable microneedle array and any other components forming a final formulation.

The kit may also comprise instructions for use.

The invention will now be further described by way of Examples, which are meant to serve to assist one of ordinary skill in the art in carrying out the invention and are not intended in any way to limit the scope of the invention.

### EXAMPLES

### Example 1 - Microneedle preparation

Microneedle arrays were prepared by the method shown schematically Fig. 1.

A master silicon microneedle array was manufactured by a silicon wet etching method as described in US2007/0134829A1 and Wilke et al. (2005 Microelectronics Journal 36:650-656). Negative microneedle moulds were made using the master silicon microneedle array by pouring liquid PDMS (polydimethylsiloxane) over the silicon array, curing at an elevated temperature (e.g. 100°C for one hour), cooling and then peeling off the flexible PDMS mould from the master silicon array.

Moulds were then cleaned in deionised water using ultrasonic bath for 20 min. Clean moulds were placed into a beaker and submerged under deionised water. The beaker was placed in a dessicator and vacuumed using water-jet pump for 20 min. The beaker with moulds was then placed in refrigerator to cool water to 4-8 °C.

Set of formulations was prepared as given in Table 1.

**Table 1. Examples of formulations used for manufacturing of microneedles**

| | **Composition/% (w/v)*** | | | | |
|---|---|---|---|---|---|
| **Formulation** | **Trehalose** | **Sucrose** | **PVA** | **PVP** | **Tween 80** |
| **1** | 50 | | | | |
| **2** | 50 | | | | 0.05 |
| **3** | 25 | 25 | | | |
| **4** | 25 | | 7.5 | | |
| **5** | | | | 50 | |
| **6** | | | 15 | | |
| **7** | | 50 | | | |

| | | | | | |
|---|---|---|---|---|---|
| *Water is used as a solvent in all formulations except for PVP where 96% ethanol was used. Methylene blue or Congo red dyes may be added for visualization | | | | | |

Formulations were thoroughly mixed and filled into PTFE tubing connected to a HPLC pump at one end and to a silicon capillary to the other end (100 µm ID).

PDMS moulds were taken from the beaker and excess water was removed from the surface by scrapping the surface with a steel blade leaving water only in the needle cavities. Flow of the formulation was set to 1 µL/min. A drop of formulation was delivered directly on top of each needle cavity. A 10X magnifier was used to aid visualisation of this process. Volume of the delivered formulation may vary depending on the type of the mould and concentration of the formulation, however in most cases it was between 15 and 80 nL per cavity.

After delivery of the formulation, the moulds were placed in a dessicator in the presence of silica gel. Moulds were left in dessicator for 5 hrs to fully dry.

A rectangular piece of 3M(TM) Single Coated Polyester Medical Tape 1516 with the dimensions exceeding the dimensions of the mould for 5mm at each side was cut and pressed on top of the mould. A gentle pressure was applied using finger tip. Microneedles were then demoulded by peeling off the adhesive tape and placed in the dessicator for storage. Fig. 4 A1-A2 shows the microneedle array and an individual needle produced using formulation 4 with the addition of methylene blue. This demonstrates the general method for making the uniform array of microneedles in which the whole needle body and the supporting disk are made of the same material.

### Example 2 - making a microneedle array by spray-filling the moulds with solvent

Microneedle moulds were prepared as described in Example 1. Moulds were then cleaned in deionised water using ultrasonic bath for 20min. Clean PDMS moulds were filled with deionised water cooled to 4-8°C by spraying water directly into moulds. Water was dispersed using a Schlick nozzle 970 S8 fitted with a 0.5mm bore. Nozzle opening was put at position 2; inlet air pressure was 0.25bars; water flow was set to 10mL/min. The nozzle to mould distance was 3.5cm. The moulds were passed under the atomised spray two times. The duration of spraying varied and in most cases was below 1 second. The making of microneedles and demoulding was further performed as described in Example 1.This demonstrates the possibility to prefill needle cavities of the mould by spray - filling thus making the process simpler and more scalable.

### Example 3 - making a microneedle array using an automated micro-volume dispensing robot.

Microneedle moulds were prepared and filled with water as described in Example 1. Formulation 1 containing methylene blue was then prepared (Table 1). Automated micro-volume dispensing machine with robotic arm movable in three dimensions was used to dispose 20nL of formulation onto each well of 12x12 microneedles/array (280µm tall needles). Thus, the total nominal volume of disposed formulation totals 2.88µL per array.

Moulds with formulation were then dried and microneedle arrays further delivered as described in Example 1.

To asses the precision of the automated machine used for dispensing the formulation onto moulds, prepared microneedle arrays were further examined. Each microneedle array was dissolved in 0.5mL of water and absorption at λ=655 nm was measured to give the total volume of the formulation delivered onto each array. The results show that the actual volume of the formulation delivered per patch was 2.90 ± 0.17µL (mean ± standard deviation, n=50).

This demonstrates the scalability and potential of the microneedle making process to be fully automated using readily available micro volume dispensing machines.

### Example 4 - Heterogeneous microneedle array preparation

Microneedle moulds were prepared and filled with water as described in Example 1. Formulation 1 containing methylene blue and formulation 3 containing Congo red were then prepared (Table 1). Formulation 1 was filled into PTFE tubing and connected to the first HPLC channel and formulation 2 filled into other tubing and connected to the second HPLC channel. Formulation 1 was then delivered on top of one half of the cavities followed by formulation 3 delivered to the other half of cavities. Moulds were then dried and demoulded as described in Example 1. Figure 4 B1-B2 show an example of microneedle array produced by the described method. This demonstrates the possibility to use the method for making heterogeneous microneedle arrays containing two or more subsets of microneedles made of different materials.

### Example 5 - Preparation of microneedle arrays with the active substance concentrated in only one part of the microneedle

Microneedle moulds were prepared and filled with water as described in Example 1. Formulation 4 containing Congo red dye was prepared, filled into PTFE tubing and connected to HPLC pump. Small volume (approx. 5 nL) was delivered on top of each needle cavity and brought in the contact with the water in the wells. The moulds were then dried in the dessicator for 5 hrs to give the dry formulation concentrated in the tip part of the microneedles. Moulds were then spray-filled with 96% ethanol cooled at -20 °C to fill the rest of the cavity volume. Excess ethanol was removed from the surface using sharp blade. Formulation 5 was then individually delivered on top of each cavity. Moulds were then dried overnight under vacuum and demoulded as described in Example 1. Fig. 4 Cl-C2 shows an example of microneedles produced by described method.This demonstrates the modification of the main method using which microneedles with the active substance concentrated in only one part of the microneedle can be prepared.

### Example 6 - Stability of vaccine components embedded in microneedles

Formulations 1 and 4 were prepared. MVA virus coding for the red fluorescent protein (MVA-RFP) was formulated in formulation 4 at a starting concentration of 10⁹ pfu/mL. Lysozyme from chicken egg white was formulated in the same formulation at a concentration of 100 mg/mL. Adenovirus (AdV) encoding mCherry protein (AdV-mCherry) was formulated in formulation 1 at the concentration of 2 x 10⁹ pfu/mL. FITC-Na was added in all the solutions at the concentration of 1 mg/mL to enable precise quantification of the amount of formulation delivered onto each individual mould. Microneedle arrays containing test components were prepared as per Example 1, sealed into individual glass vials in the presence of dessicant and kept at ambient temperature for up to 14 days. At sampling points samples were taken and frozen at -80 °C. Following 14 days incubation samples were tested for viral survival (AdV and MVA) or enzyme activity (lysozyme).

Survival of AdV and MVA expressing fluorescent proteins was measured using flow cytometry. Arrays of microneedles were dissolved in cell culture medium at ambient temperature. DF-1 (for MVA-RFP) or HEK293A (for AdV-mCherry) cells, grown under standard conditions, were infected with virus solutions and left overnight in CO₂ incubator. After 24hrs cells were harvested and infection rate was calculated by measuring fluorescence of infected cells expressing RFP or mCherry proteins using LSRII flow cytometer (Becton-Dickinson). Survival rate was calculated from standard curve using samples of known titer (in PFU/mL units) and was expressed as log PFU_{eq}/mL units (logarithmic value of plaque forming unit equivalents per mL).

Lysozyme activity was measured by standard turbidimetric assay using *Micrococcus lysodeikticus* cells.

Fig. 5 shows results of virus survival or enzymatic activity vs. time of incubation for the above described samples. It can be observed that both AdV and MVA viruses are well preserved in the microneedles with only minor titer loss while activity of lysozyme was fully preserved.

This demonstrates that various potential vaccine components including live viral vectors can be efficiently stabilized in dried microneedles using described methods.

### Example 7 - Kinetics of dissolution of microneedles ex vivo

Kinetics of dissolution of microneedles was performed using cadaver pig skin. Arrays of 500µm tall microneedles were prepared as described in Example 3 with needles' tips made of formulation 1 with the addition of Congo red dye and needles' bases made of formulation 5 with the addition of methylene blue dye. Following drying arrays were applied in vitro onto previously shaved pig skin and left for 1 s, 10 min or 60 min in the skin at 37°C. After taking the arrays off the skin both skin and arrays were imaged using light microscope. Fig. 6 shows images of microneedles and skin at respective time points.

This demonstrates that microneedles made by described methods efficiently penetrate the skin and deliver the substances embedded within the microneedle body into the skin within relatively short periods of exposure.

### Example 8 - Application of microneedles for delivery of live viral vectors ex vivo

For the skin transfection studies microneedle arrays with either 280µm or 500µm needles prepared as described in either Example 1 or Example 3 were used. AdV and MVA viruses expressing β-galactosidase were embedded in the needles at the approximate concentration of 1.5x10⁴ pfu per needle.

Freshly excised pig skin was collected and used for transfection essentially as described in Pearton et al. (2008 Pharm Res 25(2): 407-16). Arrays were left on skin for 18-24 hrs before fixation and staining. Skin was then visualized using light microscope. Fig. 7 shows successful transfection of pig skin with AdV and MVA viruses embedded in microneedles.

This demonstrates that (a) live viral vectors can be efficiently stabilized within microneedles made by described methods, (b) microneedles can penetrate the skin and deliver vectors into the skin (c) delivered vectors can infect target skin cells resulting in the expression of target proteins.

### Example 9 - Application of microneedles for delivery of vaccine in vivo

To demonstrate one example of *in vivo* utility of these dissolvable microneedle arrays, microneedle arrays with either 280µm or 25x 500µm needles, prepared as described in Example 1, were used. The 280µm arrays contained 100 microneedles, the 500µm arrays contained 25 microneedles. The vaccine antigen, tetanus toxoid, was formulated with Formulation 4 (Table 1) and embedded in the microneedle moulds at the approximate concentration of 3Lf per array. Female C57BL/6 mice were anaesthetised and microneedle arrays were applied to each ear and remained in place overnight. As controls, the tetanus toxoid in formulation 4 (labelled "TetTox in T/P ID in Figure 8) or antigen in PBS (labelled "TetTox in PBS ID in Figure 8) were injected, using a needle-and-syringe, intradermally (ID). Figure 8 demonstrates that vaccination using antigen incorporated in dissolvable microneedles induces equivalent antibody titres to administering liquid vaccine intradermally, as measured by the endpoint antibody titre. This demonstrates that dissolvable microneedles fabricated as described here can be successfully used to deliver vaccine to the living body and successfully induce an immune response.

As a second example, microneedle arrays with either 280µm or 500µm needles prepared as described in the previous example with tetanus toxoid, using Formulation 1, were used. AdV virus expressing *Plasmodium yoelii* MSP antigen, termed AdV-MSP (Draper et al (2009) Cell Host Microbe 5, 95-105 and (2008) Nat Med 14, 819-21) were embedded in the needles at the approximate concentration of 5 x 10⁹ virus particles per array. Female C57BL/6 mice were anaesthetised and microneedle arrays were applied to each ear and remained in place overnight. As a control, AdV-MSP in PBS was injected using a needle-and-syringe, intradermally (ID). Figure 9 demonstrates that vaccination using antigen incorporated in dissolvable microneedles induces equivalent anti-MSP antibody titres to administering liquid vaccine intradermally, as measured by the endpoint antibody titre. This demonstrates that dissolvable microneedles fabricated as described here can be successfully used to deliver a live vaccine to a naive and primed immune system in the living body and successfully induce an immune response.

As a third example of *in vivo* utility of these dissolvable microneedle arrays,, microneedle arrays with 500µm needles, prepared as described in the previous example with tetanus toxoid, were used to deliver seasonal, trivalent inactivated influenza virus vaccine ('TIV') to mice and induce and boost an immune response. Clinically available seasonal TIV, containing the vaccine antigens recommended for the 2010/2011 northern hemisphere vaccination campaign were embedded in the microneedles at the concentration of 0.15µg of hemagluttinin antigen (HA) from each strain per array, representing 1% of the full human dose. Female BALB/c mice were anaesthetised and microneedle arrays were applied to each ear and remained in place overnight. As a control, TIV was injected, using a needle-and-syringe by the intramuscular route (IM) at a dose of 10% of the full human dose, equivalent to 1.5µg HA from each strain. All mice were boosted with the same vaccine regime on day 28 post-prime. Figure 10 demonstrates that vaccination using 5-fold lower antigen dose incorporated in dissolvable microneedles induces equivalent antibody titres to administering liquid vaccine IM, as measured by the endpoint antibody titre to the vaccine 4 weeks after the first immunization and 2 weeks after the second immunization. This demonstrates that dissolvable microneedles fabricated as described here can be successfully used to deliver a 'dose-sparing' level of antigen to a naive and primed immune system in the living body and successfully induce and boost an immune response.

## Claims

1. A method for fabricating a microneedle using a mould (2) having a needle-forming cavity, **characterised in that** the method comprises the steps of (a) at least partially pre-filling the needle-forming cavity with a solvent (1), (b) applying a microneedle-forming composition (3) to the needle-forming cavity such that the composition is brought in contact with the solvent (1), (c) allowing the solvent and microneedle-forming composition to mix as a result of diffusion, (d) removing the solvent and (e) demoulding the microneedle.

2. A method according to claim 1, for fabricating a microneedle with a needle body formed of two composition materials, which comprises the following steps:
(a) at least partially pre-filling the needle-forming cavity with a first solvent,
(b) applying a first microneedle-forming composition to the needle-forming cavity such that the composition is brought in contact with the solvent, wherein the first microneedle-forming composition is applied in an amount to partially fill the needle cavity following solvent removal;
(c) allowing the first solvent and first microneedle-forming composition to mix as a result of diffusion;
(d) removing the first solvent;
(e) at least partially filling the remaining needle-forming cavity with a second solvent,
(f) applying a second microneedle-forming composition to the needle-forming cavity such that the composition is brought in contact with the second solvent;
(g) allowing the second solvent and second microneedle-forming composition to mix as a result of diffusion;
(h) removing the second solvent; and
(i) demoulding the microneedle,
thereby forming a microneedle with a body made of two different composition materials.

3. A method according to any preceding claim, wherein the microneedle-forming composition forms a dissolvable material following drying, such that when the microneedle is applied to the skin, or other tissue, of a subject it dissolves.

4. A method according to claim 3, wherein the microneedle-forming composition comprises an active substance, such that when the microneedle dissolves upon application to the body, the active substance is delivered into the underlying tissue of the subject.

5. A method according to claim 2, wherein the first microneedle-forming composition material forms a microneedle tip with high mechanical strength and second microneedle-forming composition forms a below-tip portion of low mechanical strength.

6. A method according to any preceding claim for forming a microneedle array, by using a mould having a plurality of needle-forming cavities, such that a plurality of microneedles are fabricated.

7. A method according to claim 2, for forming a microneedle array, by using a mould having a plurality of needle-forming cavities, wherein the first and/or second microneedle-forming compositions are delivered successively or simultaneously on different microneedle cavities of the same mould thus forming a heterogeneous microneedle array.

8. A method according to any preceding claim, where microneedle is demoulded by adhering to an adhesive surface applied on top of the filled mould and pulling microneedles out of the mould.

## Patentansprüche

1. Verfahren zur Herstellung einer Mikronadel mithilfe einer Gussform (2) mit einem nadelformenden Hohlraum, **dadurch gekennzeichnet, dass** das Verfahren Folgendes umfasst: die Schritte (a) zumindest teilweises Vorbefüllen des nadelformenden Hohlraums mit einem Lösungsmittel (1), (b) Aufbringen einer mikronadelformenden Zusammensetzung (3) in den nadelformenden Hohlraum derart, dass die Zusammensetzung mit dem Lösungsmittel (1) in Kontakt gebracht wird, (c) Zulassen der Vermischung des Lösungsmittels und der mikronadelformenden Zusammensetzung infolge von Diffusion, (d) Entfernen des Lösungsmittels und (e) Entformen der Mikronadel.

2. Verfahren nach Anspruch 1 zur Herstellung einer Mikronadel mit einem aus zwei Zusammensetzungsmaterialien gebildeten Nadelkörper, welches die folgenden Schritte umfasst:
(a) zumindest teilweises Vorbefüllen des nadelformenden Hohlraums mit einem ersten Lösungsmittel,
(b) Aufbringen einer ersten mikronadelformenden Zusammensetzung in den nadelformenden Hohlraum derart, dass die Zusammensetzung mit dem Lösungsmittel in Kontakt gebracht wird, wobei die erste mikronadelformende Zusammensetzung in einer Menge aufgebracht wird, dass sie nach Entfernen des Lösungsmittels teilweise den Nadelhohlraum ausfüllt;
(c) Zulassen der Vermischung des ersten Lösungsmittels und der ersten mikronadelformenden Zusammensetzung infolge von Diffusion;
(d) Entfernen des ersten Lösungsmittels;
(e) zumindest teilweises Befüllen des verbleibenden nadelformenden Hohlraums mit einem zweiten Lösungsmittel,
(f) Aufbringen einer zweiten mikronadelformenden Zusammensetzung in den nadelformenden Hohlraum derart, dass die Zusammensetzung mit dem zweiten Lösungsmittel in Kontakt gebracht wird;
(g) Zulassen der Vermischung des zweiten Lösungsmittels und der zweiten mikronadelformenden Zusammensetzung infolge von Diffusion;
(h) Entfernen des zweiten Lösungsmittels und
(i) Entformen der Mikronadel,
dadurch Herausbilden einer Mikronadel mit einem aus zwei verschiedenen Zusammensetzungsmaterialien gefertigten Körper.

3. Verfahren nach einem der vorhergehenden Ansprüche, wobei die mikronadelformende Zusammensetzung ein nach dem Trocknen lösbares Material bildet derart, dass es sich auflöst, wenn die Mikronadel auf der Haut oder anderem Gewebe einer Person angewendet wird.

4. Verfahren nach Anspruch 3, wobei die mikronadelformende Zusammensetzung einen Wirkstoff umfasst derart, dass, wenn sich die Mikronadel bei Anwendung an dem Körper auflöst, der Wirkstoff in das darunter liegende Gewebe der Person geleitet wird.

5. Verfahren nach Anspruch 2, wobei das erste mikronadelformende Zusammensetzungsmaterial eine Mikronadelspitze mit hoher mechanischer Beständigkeit und die zweite mikronadelformende Zusammensetzung einen Abschnitt unterhalb der Spitze mit geringer mechanischer Beständigkeit bildet.

6. Verfahren nach einem der vorhergehenden Ansprüche zum Ausbilden einer Mikronadelanordnung mithilfe einer Gussform mit einer Vielzahl von nadelformenden Hohlräumen derart, dass eine Vielzahl von Mikronadeln hergestellt werden.

7. Verfahren nach Anspruch 2 zum Ausbilden einer Mikronadelanordnung mithilfe einer Gussform mit einer Vielzahl von nadelformenden Hohlräumen, wobei die erste und/oder zweite mikronadelformende Zusammensetzung nacheinander oder gleichzeitig auf verschiedene Mikronadelhohlräume derselben Gussform geleitet werden bzw. wird und somit eine heterogene Mikronadelanordnung gebildet wird.

8. Verfahren nach einem der vorhergehenden Ansprüche, wobei die Mikronadel durch Anhaften an einer auf der Oberseite der befüllten Gussform angebrachten Haftfläche und durch Ziehen der Mikronadeln aus der Gussform entformt wird.

## Revendications

1. Procédé pour fabriquer une micro-aiguille à l'aide d'un moule (2) ayant une cavité de formation d'aiguille, **caractérisé en ce que** le procédé comprend les étapes consistant à : (a) préremplir au moins partiellement la cavité de formation d'aiguille avec un solvant (1) ; (b) appliquer une composition de formation de micro-aiguille (3) à la cavité de formation d'aiguille de telle sorte que la composition soit mise en contact avec le solvant (1) ; (c) permettre au solvant et à la composition de formation de micro-aiguille de se mélanger à la suite d'une diffusion ; (d) éliminer le solvant et (e) démouler la micro-aiguille.

2. Procédé selon la revendication 1 pour fabriquer une micro-aiguille avec un corps d'aiguille formé de deux matériaux de composition, qui comprend les étapes suivantes consistant à :
(a) préremplir au moins partiellement la cavité de formation d'aiguille avec un premier solvant ;
(b) appliquer une première composition de formation de micro-aiguille à la cavité de formation d'aiguille de telle sorte que la composition soit mise en contact avec le solvant, dans lequel la première composition de formation de micro-aiguille est appliquée en une certaine quantité de sorte à remplir partiellement la cavité d'aiguille après l'élimination du solvant ;
(c) permettre au premier solvant et à la première composition de formation de micro-aiguille de se mélanger à la suite d'une diffusion ;
(d) éliminer le premier solvant ;
(e) remplir au moins partiellement la cavité de formation d'aiguille restante avec un second solvant ;
(f) appliquer une seconde composition de formation de micro-aiguille à la cavité de formation d'aiguille de telle sorte que la composition soit mise en contact avec le second solvant ;
(g) permettre au second solvant et à la seconde composition de formation de micro-aiguille de se mélanger à la suite d'une diffusion ;
(h) éliminer le second solvant ; et
(i) démouler la micro-aiguille,
ce qui permet de former une micro-aiguille avec un corps composé de deux différents matériaux de composition.

3. Procédé selon l'une quelconque des revendications précédentes, dans lequel la composition de formation de micro-aiguille forme un matériau soluble après séchage de telle sorte que, lorsque la micro-aiguille est appliquée sur la peau, ou un autre tissu, d'un sujet, elle se dissolve.

4. Procédé selon la revendication 3, dans lequel la composition de formation de micro-aiguille comprend une substance active de telle sorte que, lorsque la micro-aiguille se dissout lors de son application sur le corps, la substance active est administrée dans le tissu sous-jacent du sujet.

5. Procédé selon la revendication 2, dans lequel la première composition de formation de micro-aiguille forme une pointe de micro-aiguille ayant une forte résistance mécanique et la seconde composition de formation de micro-aiguille forme une partie sous la pointe ayant une faible résistance mécanique.

6. Procédé selon l'une quelconque des revendications précédentes pour former un ensemble de micro-aiguilles, à l'aide d'un moule ayant une pluralité de cavités de formation d'aiguille de telle sorte qu'une pluralité de micro-aiguilles soient fabriquées.

7. Procédé selon la revendication 2, pour former un ensemble de micro-aiguilles, à l'aide d'un moule ayant une pluralité de cavités de formation d'aiguille, dans lequel la première et/ou la seconde composition de formation de micro-aiguille est/sont administrée(s) successivement ou en même temps sur différentes cavités de micro-aiguille du même moule, ce qui permet de former un ensemble de micro-aiguilles hétérogène.

8. Procédé selon l'une quelconque des revendications précédentes, dans lequel une micro-aiguille est démoulée en la faisant adhérer à une surface adhésive appliquée sur la partie supérieure du moule rempli et en retirant des micro-aiguilles du moule.
